# EUROPÄISCHE PATENTSCHRIFT

(11) **EP 1 737 946 B1**
(45) Veröffentlichungstag und Bekanntmachung des Hinweises auf die Patenterteilung: **20.10.2010**
(21) Anmeldenummer: 04725918.9
(22) Anmeldetag: 06.04.2004
(51) Int. Cl.: C12N 1/20, C12N 9/88, C12N 1/38

(54) **VERFAHREN ZUR KULTIVIERUNG DES NITRILHYDRATASE-PRODUZIERENDEN STAMMES RODOCOCCUS RHODOCHROUS M33**
METHOD FOR CULTURING THE NITRILE HYDRATASE-PRODUCING STRAIN RODOCOCCUS RHODOCHROUS M33
PROCEDE DE MISE EN CULTURE DE LA SOUCHE DE RHODOCOCCUS RHODOCROUS M33 PRODUISANT LA NITRILHYDRATASE

(43) Veröffentlichungstag der Anmeldung: 03.01.2007
(73) Patentinhaber: Ashland Licensing and Intellectual Property LLC, Dublin, OH 43017 (US)
(72) Erfinder: LORENZ, Josef, 47809 Krefeld (DE); WORONIN, S., P., Saratov, 410028 (RU); KOSOLIN, S., W., Saratov, 410078 (RU); SINGIRZEV, I., N., Saratow, 410033 (RU); DEBABOV, V., G., Moskau 115409 (RU); YANENKO, A., S., Moskau, 121293 (RU)
(74) Vertreter: Bülle, Jan
(86) Internationale Anmeldenummer: PCT/EP2004/003611
(87) Internationale Veröffentlichungsnummer: WO 2005/108553

(56) Entgegenhaltungen:
- EP-A- 1 142 997
- WO-A-02/081659
- DE-A- 10 315 376
- US-A- 5 827 699
- HONDA, HIROYUKI ET AL: "High cell density culture of Rhodococcus rhodochrous by pH-Stat feeding and dibenzothiophene degradation." JOURNAL OF FERMENTATION AND BIOENGINEERING, VOL. 85, NO. 3, PP. 334-338. PRINT. CODEN: JFBIEX. ISSN: 0922-338X., 1998, XP002311395
- LEONOVA (POGORELOVA), TATYANA E. ET AL: "Nitrile hydratase of Rhodococcus: Optimization of synthesis in cells and industrial applications for acrylamide production." APPLIED BIOCHEMISTRY AND BIOTECHNOLOGY, (JULY-SEPTEMBER, 2000) VOL. 88, NO. 1-3, PP. 231-241. PRINT. CODEN: ABIBDL. ISSN: 0273-2289., 2000, XP009027439
- BU-YON KIM, JONG-CHUL KIM, HYUNE-HWAN LEE AND HYUNG-HWAN HYUN: "Fed-Batch Fermentation for Production of Nitrile Hydratase by Rhodococcus rhodochrous M33" BIOTECHNOL. BIOPROCESS ENG., Bd. 6, 2001, Seiten 11-17, XP009041673 in der Anmeldung erwähnt

## Beschreibung

Die Erfindung betrifft ein biotechnologisches Verfahren zur Kultivierung des Nitrilhydratase-produzierenden Stammes *Rhodococcus rhodochrous* M33 sowie ein im Verfahren verwendetes Kultivierungsmedium zur Steigerung der Biomassenausbeute mit hoher Nitrilhydratase-Aktivität.

Die Entwicklung industrieller biotechnologischer Produktionsverfahren für chemische Verbindungen führte zu großem Interesse an Mikroorganismen mit speziellen Enzymen, die für ausgewählte biokatalytische Reaktionen einsetzbar sind. Ein Beispiel hierfür ist das biotechnologische Produktionsverfahren zur Herstellung von Amiden, das auf der Fähigkeit einiger Mikroorganismen basiert, Nitrilhydratase zu synthetisieren, einem Enzym, das die Umwandlung von Nitrilen organischer Säuren in die entsprechenden Amide katalysiert. Besonderes Interesse gilt hierbei insbesondere Bakterienstämmen, die Acrylnitril in Acrylamid transformieren können.

Eine wichtige Rolle bei der Produktion der katalytisch aktiven Biomasse bzw. des Biokatalysators spielt das Verfahren zur Kultivierung der jeweiligen Mikroorganismen. Üblicherweise ist die Erhöhung der Enzymaktivitäts-Ausbeute pro Volumeneinheit der Kulturbrühe (Gesamtaktivität in U/ml) auf zwei Wegen möglich:
1. Aktivierung der Enzym-Synthese der (Bakterien-) Zelle, d.h. Erhöhung der spezifischen Enzym-Aktivität (U/mg Biotrockenmasse)
2. Steigerung der Ausbeute an aktiven Zellen (aktive Biomasse) pro Volumeneinheit der Kulturbrühe

In der GB 2 087 926 A und der dort zitierten Patentliteratur wird u.a. das Kultivierungsverfahren für den Stamm *Corynebacterium* sp. N-774 (Hinterlegungsnummer Fermentation Research Institute FERM No. 4446) beschrieben. Der Stamm, der über eine Nitrilhydratase verfügt, wird in einem Mineralmedium kultiviert, das eine wasserlösliche Eisenverbindung in Mengen von mindestens 0,2 mg/l enthält sowie 1 Gew.-% Caseinhydrolysate (casamino acids). Die Nachteile dieses Verfahrens sind die hohen Kosten der Caseinhydrolysate und die erzielte niedrige Nitrilhydrataseaktivität: spezifische Aktivität - 53,3 U/mg, Biotrockenmasse - 5,66 mg/ml, Gesamtaktivität - 301,7 U/ml.

Es ist bekannt, dass Nitrile und Amide organischer Säuren, insbesondere der Propion-und Isobuttersäure, zur Induktion der Nitrilhydratase führen. Der US 4 555 487 kann diesbezüglich entnommen werden, dass die Zugabe solcher Verbindungen, sogenannte enzyminduzierende Mittel bzw. Induktoren, in das Kultivierungsmedium der Stämme *Pseudomonas chlororaphis* Stamm B23 (FERM BP-187) und *Pseudomonas* sp. Stamm PS 1 (FERM BP-188) es möglich macht, Biomasse (5,36 g/l Biotrockenmasse) mit einer spezifischen Aktivität von 62,65 U/mg und eine Gesamtaktivität von 335,8 U/ml zu erhalten.
Die Nachteile dieses Verfahrens bestehen in der Nutzung von kostenintensiven Induktoren und einer niedrigen Nitrilhydratase-Aktivität der gewonnenen Zellen.

Das in der EP 0 115 781 beschriebene Kulturmedium zur Züchtung der Stämme *Pseudomonas chlororaphis* Stamm B23 (FERM BP-187) und *Pseudomonas* sp. Stamm PS-1 (FERM BP-188) enthält außer den z.B. in der US 4 555 487 beschriebenen Induktoren eine oder mehrere α - Aminosäuren als Mittel zur Erhöhung der Enzymaktivität. So werden α - Aminosäuren wie z.B. Cystin, Cystein, Alanin, Valin, Methionin etc. in einer Konzentration im Bereich von 0,1 bis 10,0 g/l eingesetzt. Dennoch übersteigt die spezifische Aktivität der auf die Weise gewonnenen Biomasse nicht den Wert 105,7 U/mg, bzw. die Gesamtaktivität von 428,1 U/ml.

Eine höhere Nitrilhydratase-Aktivität wurde bei der Züchtung des Stammes Rhodococ*cus rhodochrous* M33 erreicht, die in der DE 44 80 132 beschrieben ist. Dort wurde gezeigt, dass es bei der Stammkultivierung im synthetischen Medium mit Glucose (Konzentration 5 g/l) möglich ist, Zellen mit einer spezifischen Aktivität bis zu 200 U/mg sowie eine Gesamtaktivität bis 360 U/ml zu erhalten. Ein wesentlicher Vorteil des vorgeschlagenen Verfahrens ist die Einfachheit des Mediums, das keine kostspieligen Komponenten enthält. Jedoch übersteigt die Gesamtaktivität bei der Erhöhung der Glucose-Konzentration auf 20 g/l nicht den Wert von 1368 U/ml.

Ein technisch adäquates Verfahren ist das Verfahren zur Kultivierung des Stammes *Rhodococcus rhodochrous,* Stamm J1 (FERM BP-1478), das in der EP 0 362 829 beschrieben wird. Im Medium, das einen preiswerten Induktor - Harnstoff (Konzentration 15 g/l) und Verbindungen des bivalenten Kobalts enthält - ermöglicht das vorgeschlagene Kultivierungsverfahren, Zellen mit einer spezifischen Aktivität von 578 U/mg zu erhalten. Zur Steigerung der Zellausbeute (Biomasseausbeute) werden zusätzlich Pepton und Hefeextrakt dem Medium beigemischt. Jedoch übersteigen die Werte der Gesamtaktivität nicht 2480 U/ml, weil die Biomasseausbeute nur 4,3 g/l beträgt. Beim "upscaling" des Kultivierungsprozesses unter industriellen Bedingungen gelang es die Zellausbeute auf 28 g/l zu erhöhen; dabei sank die spezifische Nitrilhydratase-Aktivität auf 76 U/mg, die Gesamtaktivität sank auf 2100 U/ml (Yamada H., Kobayashi M., "Nitrile Hydratase and Application to Industrial Production of Acrylamide", Biosci. Biotech. Biochem., 60 (9), 1391-1400, 1996.

In der Literatur (Kim B-Y., Kim J-C., Lee H-H., Hyun H-H., "Fed-batch Fermentation for Production of Nitrile Hydratase by Rhodococcus rhodochrous M33", Biotechnol. Bioprocess Eng., 6: 11-17, 2001) ist ein weiteres Kultivierungsverfahren für den Stamm *Rhodococcus rhodochrous* M33 beschrieben. Hierbei handelt es sich um eine Glucoselimitierte fed batch-Fermentation in einem 5 1-Fermenter mit einem rein synthetischen Kultivierungsmedium, bestehend aus KH₂PO₄, K₂HPO₄, FeSO₄ x 7 H₂O, EDTA-2Na, MgSO₄ x 7 H₂O, NaCl, CoCl₂ x 6 H₂O, Harnstoff und Glucose. Durch konstante Dosierung einer 40 %-igen Glucoselösung und drei periodischen CoCl₂ x 6 H₂O-Zugaben (0,01 g/l) in die Glucoselösung wurden eine Zellausbeute von 24 g/l und eine spezifische Nitrilhydratase-Aktivität von 120 U/mg erreicht. Dennoch übersteigt die Gesamtaktivität nach einer relativ langen Fermentationsdauer von ungefähr 115 Stunden nicht den Wert von 2880 U/ml.

Es besteht somit weiterhin ein Bedürfnis nach biotechnologischen Verfahren zur Kultivierung von Nitrilhydratase-produzierenden Stämmen, die unter industriellen Bedingungen eine Steigerung der Biomasseausbeute mit hoher Nitrilhydratase-Aktivität ermöglichen.

Der vorliegenden Erfindung liegt daher die Aufgabe zugrunde, ein solches Verfahren aufzufinden.

Überraschenderweise konnte gefunden werden, dass ein Verfahren zur Kultivierung des Nitrilhydratase-produzierenden Stammes *Rhodococcus rhodochrous* M33, der unter der Hinterlegungsbezeichnung DSM 14230 bei der DSMZ, Deutsche Sammlung von Mikroorganismen und Zellkulturen GmbH, Marschroder Weg 1b, D-38124 Braunschweig, Deutschland, hinterlegt ist, die erfindungsgemäße Aufgabe löst, wenn zur Kultivierung ein Kultivierungsmedium verwendet wird, das auf einem 12 bis 60 mM Phosphat-Puffer basiert und den Bedarf der Zellen an Phosphor deckt sowie den pH-Wert während der Kultivierung im Bereich von 5,5 bis 9,0 aufrecht erhält und in dem nach dem Verbrauch der vorgelegten Glucosemenge Essigsäure als neue Kohlenstoffquelle zudosiert wird wobei dem Kultivierungsmedium Harnstoff und CoCl₂ x 6 H₂O zudosiert werden.

Erfindungsgemäß wird ein Kultivierurigsmedium eingesetzt, das den Bedarf der Zellen an Phosphor deckt und es ermöglicht, den pH-Wert während der Kultivierung im Bereich von 5,5 bis 9,0 aufrecht zu erhalten. Vorzugsweise enthält ein solches Kultivierungsmedium Maisextrakt als zusätzlichen Wachstumsfaktor.

Besonders bevorzugt wird im erfindungsgemäßen Verfahren ein Maisextrakt im Kultivierungsmittel als zusätzlicher Wachstumsfaktor eingesetzt, der z.B. in Form von Maisquellwasser bzw. Corn Steep-Lösung in einer Konzentration von beispielsweise 1 bis 10 g/l zugesetzt wird. Als Maisextrakte sind alle Maisquellwässer einsetzbar, die die mittels üblichen Quellprozeß in Lösung gegangenen Inhaltsstoffe des Maiskorns in konzentrierter Form enthalten. Bevorzugt werden erfindungsgemäß Maisquellwässer verwendet, die unter der Bezeichnung Cerestar 15840 von der Firma Cerestar Deutschland GmbH, Krefeld vertrieben werden.

Das im erfindungsgemäßen Verfahren einsetzbare Kultivierungsmedium (nachfolgend SI genannt) hat vorzugsweise die Zusammensetzung (in g/l):
a.) 7,9 g/l Na₂HPO₄ x 12 H₂O
b.) 1,8 g/l KH₂PO₄
c.) 0,02 bis 0,04 g/l CoCl₂ x 6 H₂O
d.) 1,0 g/l MgSO₄ x 7 H₂O
e.) 0 bis 10 g/l Maisextrakt
f.) 20 bis 90 g/l Glucose
g.) 4 bis 20 g/l Harnstoff

Insbesondere wird ein solches Kultivierungsmedium verwendet, dem Maisextrakt in einer Konzentration von 1 bis 10 g/l, vorzugsweise von 2 bis 8 g/l und besonders bevorzugt von 2 bis 6 g/l zugesetzt ist. Ganz besonders bevorzugt kann der Maisextrakt in einer Konzentration von 4 g/l im Kultivierungsmedium verwendet werden.

Die Grundlage dieses Kultivierungsmediums ist ein 12 bis 60 mM Phosphat-Puffer, der den Bedarf der Zellen an Phosphor deckt und es ermöglicht, den pH-Wert während der Kultivierung im Bereich von 5,5 bis 9,0 aufrecht zu erhalten, wobei als Quellen für Kohlenstoff Glucose und Essigsäure eingesetzt werden und Essigsäure als neue zusätzliche Kohlenstoffquelle neben Glucose eingesetzt wird.
Besonders bevorzugt wird hierbei ein 35,3 mM Phosphat-Puffer im erfindungsgemäßen Verfahren verwendet.

Vorteilhaft enthält das Kultivierungsmedium, insbesondere bei der Kultivierung in Schüttelkolben 20 bis 90 g/l, vorzugsweise 20 bis 60 g/l und besonders bevorzugt 20 bis 40 g/l Glucose als Kohlenstoffquelle. Als weitere Kohlenstoffquelle kann neben Glucose Essigsäure verwendet werden, wobei vorzugsweise die Zugabe der Kohlenstoffquellen, insbesondere bei der Kultivierung in Fermentern - im Labor- bis großtechnischen Maßstab - in Form eines Zwei-Phasen-Verfahrens erfolgt:

Die erste Phase ist hierbei gekennzeichnet durch ein eingeleitetes, verstärktes Zellwachstum von *Rhodococcus rhodochrous* M33 auf Basis der im Medium vorgelegten ersten Kohlenstoffquelle, vorzugsweise 20 g/l Glucose. Nach dem Verbrauch der Glucose wird die Fermentation in der zweiten Phase als sogenannter fed-batch-Prozess, bei dem Essigsäure als Kohlenstoffquelle verwendet wird, fortgesetzt. Die kostengünstigere Essigsäure kann die für die Kultivierung verwendbare Glucose ersetzen und gewährleistet eine hohe spezifische Nitrilhydrataseaktivität und eine hohe Gesamtaktivität.

Vorteilhaft kann die Zugabe der Essigsäure auch in Form ihrer wasserlöslichen Salze erfolgen, wobei Natriumacetat als wasserlösliches Acetatsalz besonders bevorzugt ist. Das erfindungsgemäß einzusetzende wasserlösliche Acetatsalz wird in einer Ausführungsform der Erfindung nach dem Verbrauch der Glucose zudosiert, wobei die Zugabemenge des vorzugsweise zu verwendenden Natriumacetat 1 bis 4 g/l betragen kann. Eine Zugabemenge von Natriumacetat 3 g/l ist hierbei besonders bevorzugt.

In einer weiteren Ausführungsform des erfindungsgemäßen Verfahrens wird das wasserlösliche Salz der Essigsäure, vorzugsweise Natriumacetat zunächst vorgelegt. Nach Anstieg des pH-Wertes auf pH 7,5 bis 8,5 wird anschließend Essigsäure als 20 bis 50 %-ige, vorzugsweise als 30%-ige wässrige Lösung zudosiert. In vorteilhafter Weise erfolgt die Dosierung der wässrigen Essigsäurelösung über ein pH-Stat-Feeding bei pH 7.5 bis 8.5, insbesondere bei pH 7.9.

CoCl₂ x 6 H₂O und Harnstoff werden zu bestimmten Zeitpunkten während der Fermentation in geeigneter Konzentration zudosiert wobei das Cobalt als Cofaktor und der Harnstoff als Induktor dient.

Insbesondere werden zur Induktion der Nitrilhydratase dem Kultivierungsmedium 4 bis 20 g/l Harnstoff und 0,02 bis 0,04 g/l CoCl₂ x 6 H₂0 zudosiert. Vorzugsweise erfolgt die Harnstoffzugabe in der Weise, dass der Harnstoff als Induktor teilweise im Medium vorgelegt wird, insbesondere in einer Einsatzmenge von 4 g/l, und nach dem Verbrauch der vorgelegten Glucose nochmals, bevorzugt in einer Menge von 6 g/l, zudosiert wird.

Die Zugabe des Cofaktors CoCl₂ x 6 H₂0 kann erfindungsgemäß in der Weise erfolgen, dass das CoCl₂ x 6 H₂0 zu Beginn der exponentiellen Wachstumsphase, die auf der Kohlenstoffquelle Glucose basiert, dem Medium beigemischt wird, bevorzugt in einer Einsatzmenge von 0,01 g/l, und nach dem Verbrauch der Glucose nochmals, vorzugsweise in einer Einsatzmenge von 0,03 g/l, zudosiert wird.

Es wurde überraschend gefunden, dass eine Verkürzung der Fermentationsdauer erzielt werden kann, wenn das erfindungsgemäße Verfahren in der Weise geführt wird, dass CoCl₂ x 6 H₂O, vorzugsweise 0,01 g/l, zu Beginn der exponentiellen Wachstumsphase zudosiert wird.

Die Stammkultivierung des erfindungsgemäßen Verfahrens erfolgt in Schikanenkolben (Schüttelkulturen) und in Labor- und Industrie - Fermentern im Temperaturbereich 25 bis 30°C und einer Kultivierungszeit von 48 bis 170 Stunden. In einer bevorzugten Ausführungsform des erfindungsgemäßen Verfahrens wird bei der Kultivierung, insbesondere in industriellen Fermentern die Sauerstoffversorgung so gestaltet, dass der relative O₂-Partialdruck größer 30 %, vorzugsweise ≥ 50 %, ist.

Die Vorteile des erfindungsgemäßen Kultivierungsverfahren des Nitrilhydratase-produzierenden Stammes *Rhodococcus rhodochrous* M33 sind, dass unter Verwendung des erfindungsgemäßen Kultivierungsmediums, insbesondere mit Maisextrakt als zusätzlichem Wachstumsfaktor sowohl große Mengen an Nitrilhydratase-haltiger Biomasse (Ausbeute bis 39,5 g/l Biotrockenmasse), als auch eine Biomasse mit einer hohen spezifischen Nitrilhydratase-Aktivität (bis 315 U/mg) hergestellt werden kann, wobei sich eine Nitrilhydratase-Gesamtaktivität von 7169 U/ml erzielen läßt, wie den nachfolgenden Beispielen entnommen werden kann.

Im Unterschied zu den bekannten Kultivierungsverfahren für Nitrilhydrataseproduzierende Stämme zeichnet sich das erfindungsgemäß vorliegende Verfahren durch die Verwendung eines Kultivierungsmediums aus, das auf einem 12 bis 60 mM Phosphat-Puffer basiert, der den Bedarf der Zellen an Phosphor deckt sowie den pH-Wert während der Kultivierung im Bereich von 5,5 bis 9,0 aufrecht erhält und nach dem Verbrauch der vorgelegten Glucosemenge Essigsäure als neue Kohlenstoffquelle zudosiert. Außerdem können als zusätzlicher Wachstumsfaktor Maisextrakte in Form von Maisquellwasser oder Corn Steep-Lösung verwendet werden. Weiterhin ist vorteilhaft, dass im Kultivierungsmedium die für die Kultivierung verwendbare Glucose durch die kostengünstigere Essigsäure ersetzt werden kann und so eine hohe spezifische Nitrilhydrataseaktivität und hohe Gesamtaktivität des erfindungsgemäßen Verfahrens gewährleistet ist.

### Standardtest zur Messung der Nitrilhydratase-Aktivität

1 ml 2 %-ige Acrylnitril-Lösung in 10 mM Phosphat-Puffer (pH 7,5) werden mit 1 ml M33-Zellsuspension vermischt, die durch vorheriges Zentrifugieren der Kulturbrühe, Waschen und anschließendem Resuspendieren der Zellen in 10 mM Phosphat-Puffer (pH 7,5) erhalten wurde. Die Suspension enthält 0,08 bis 0,16 mg Zellen (Trockenmasse). Die Reaktion läuft 5 min bei 20°C, danach wird die Reaktion durch Zugabe von 20 µl konzentrierter HCl angehalten. Die Konzentration des gebildeten Acrylamids wird gaschromatographisch oder photometrisch bestimmt.

Die Nitrilhydratase-Aktivität wird in folgenden Einheiten angegeben :
- spezifische Aktivität - in µmol Acrylamid / min x mg Biotrockenmasse = [U/mg]
- Gesamtaktivität - in µmol Acrylamid / min x ml Kulturbrühe = [U/ml]

Die Erfindung wird anhand folgender Beispiele illustriert:

### Beispiel 1:

Schikanenkolben (250 ml) mit 50 ml Kulturivierungsmedium "SI" (Zusammensetzung in g/l: Na₂HPO₄ x 12 H₂O -7,9; KH₂PO4-1,8; CoCl₂ x 6 H₂O -0,02; MgSO₄ x 7 H₂O -1,0; Maisextrakt - 2,0; pH 7,2 ± 0,2), das Glucose (50 g/l) und Harnstoff (4 bis 20 g/l) enthält, werden mit 1 ml *Rhodococcus rhodochrous* M33-Inokulumkultur beimpft. Das Inokulum wurde innerhalb von 24 Stunden im gleichen Medium angezüchtet. Die Kultivierung wird in einem Schüttelinkubator bei 180 Umdrehungen/min (kreisförmig) und 30°C innerhalb von 96 Stunden durchgeführt. Die Ausbeute an Biomasse und die Nitrilhydratase-Aktivität der Zellen werden ermittelt. Die Ergebnisse sind in Tabelle 1 dargestellt.

**Tabelle 1**

| Harnstoff-Konzentration [g/l] | Biotrockenmasse-Ausbeute [g/l] | Nitrilhydratase-Aktivität | |
|---|---|---|---|
| | | Spezifische Aktivität [U/mg] | Gesamtaktivität [U/ml] |
| 4 | 22,3 | 62 | 1383 |
| 8 | 24,2 | 126 | 3040 |
| 12 | 24,4 | 150 | 3660 |
| 16 | 25,1 | 177 | 4453 |
| 20 | 23,0 | 173 | 3970 |

### Beispiel 2:

Schikanenkolben (250 ml) mit 50 ml Kultivierungsmedium "SI" (Zusammensetzung in g/l: Na₂HPO₄ x 12 H₂O -7,9; KH₂PO₄-1,8; CoCl₂x 6 H₂O -0,02; MgSO₄ x 7 H₂O -1,0; Harnstoff -16,0; pH 7,2 ± 0,2), das Glucose (50 g/l) und Maisextrakt (0 bis 10 g/l) enthält, werden mit 1 ml Rhodococcus rhodochrous M33-Inokulumkultur beimpft. Das Inokulum wurde innerhalb von 24 Stunden im gleichen Medium angezüchtet. Die Kultivierung wird in einem Schüttelinkubator bei 180 Umdrehungen/min (kreisförmig) und 30°C innerhalb von 96 Stunden durchgeführt. Die Ausbeute an Biomasse und die Nitrilhydratase-Aktivität der Zellen werden ermittelt und die Ergebnisse sind in Tabelle 2 dargestellt.

**Tabelle 2**

| Maisextrakt-Konzentration [g/l] | Biotrockenmasse-Ausbeute [g/l] | Nitrilhydratase-Aktivität | |
|---|---|---|---|
| | | Spezifische Aktivität, [U/mg] | Gesamtaktivität [U/ml] |
| 0* | 23,3 | 126 | 2940 |
| 1 | 23,5 | 140 | 3299 |
| 2 | 24,4 | 150 | 3660 |
| 4 | 24,9 | 172 | 4290 |
| 6 | 24,6 | 163 | 4012 |
| 8 | 24,4 | 156 | 3802 |
| 10 | 25,3 | 141 | 3562 |

| | | | |
|---|---|---|---|
| * Diesem Ansatz werden 0,01 g/l FeSO₄x 7 H₂0 zudosiert. | | | |

Aus den vorgelegten Daten ist es ersichtlich, dass die Zugabe des Maisextraktes zur Erhöhung der spezifischen und der Gesamt-Nitrilhydratase-Aktivität führt. Die optimale Konzentration des Maisextraktes beträgt 4 g/l.

### Beispiel 3:

Schikanenkolben (250 ml) mit 50 ml Kultivierungsmedium "SI" (Zusammensetzung in g/l: Na₂HPO₄ x 12 H₂O -7,9; KH₂PO₄-1,8; CoCl₂ x 6 H₂O -0,02; MgSO₄ x 7 H₂O -1,0; Harnstoff -16,0; Maisextrakt - 4,0; pH 7,2 ± 0,2) enthalten Glucose in den Konzentrationen 20 bis 90 g/l. Die Inokulation und Inkubation der Kolben werden wie in Beispiel 1 durchgeführt. Die gemessene Biomasseausbeute und die Nitrilhydratase-Aktivität der Zellen sind in der Tabelle 3 zusammengefaßt.

**Tabelle 3**

| Glucose-Konzentration [g/l] | Biotrockenmasse-Ausbeute | Inkubationszeit | Nitrilhydratase-Aktivität | |
|---|---|---|---|---|
| | | | Spezifische Aktivität [U/mg] | Gesamtaktivität [U/ml] |
| | [g/l] | [Stunden] | | |
| 20 | 10,5 | 72 | 306 | 3212 |
| 30 | 15,9 | 72 | 255 | 4051 |
| 40 | 19,2 | 96 | 223 | 4282 |
| 50 | 23,5 | 96 | 216 | 5076 |
| 60 | 27,4 | 120 | 204 | 5595 |
| 70 | 32,7 | 144 | 198 | 6465 |
| 80 | 36,4 | 166 | 191 | 6956 |
| 90 | 39,5 | 166 | 182 | 7169 |

Wie aus der Tabelle 3 folgt, erhöht sich die Zellausbeute und die Gesamt-Nitrilhydratase-Aktivität proportional der Erhöhung der Konzentration der Glucose im Kulturmedium. Dies resultiert daraus, dass alle Komponenten des Kulturmediums in nicht limitierenden Mengen vorhanden sind.

### Beispiel 4:

Ein 3 I-Laborfermenter mit 1,5 I Medium "SI" (Zusammensetzung in g/l: Na₂HPO₄ x 12 H₂O -7,9; KH₂PO₄-1,8; CoCl₂ x 6 H₂O -0,01; MgSO₄ x 7 H₂O -1,0; Harnstoff -16,0; Maisextrakt - 4,0; pH 7,2 ± 0,2), das 5 g/l Glucose enthält, wird mit 100 ml *Rhodococcus rhodochrous* M33-Inokulumkultur beimpft. Das Inokulum wurde innerhalb von 24 Stunden in einem Medium gleicher Zusammensetzung gezüchtet.

Kultivierungsbedingungen:
- Temperatur 30°C
- Rührer-Drehzah l 560 min⁻¹
- Belüftungsrate 1,5 wm (volume/volume/minute)

Nach 18 Stunden Fermentationsdauerwerden alle 2 Stunden je 1,5 g/l Glucose bis zu einer Endkonzentration von 40 g/l zudosiert. Im Ergebnis gelang es, 16,4 g/l Biotrockenmasse mit einer spezifischen Aktivität von 215 U/mg herzustellen. Die Gesamtaktivität betrug 3526 U/ml.

### Beispiel 5:

Ein 3 I-Laborfermenter mit 1,5 I Medium "SI" (Zusammensetzung in g/l: Na₂HPO₄ x 12 H₂O -7,9; KH₂PO₄-1,8; CoCl₂ x 6 H₂O -0,01; MgSO₄ x 7 H₂O-1,0; Harnstoff-4,0; Maisextrakt - 4,0; pH 7,2 ± 0,2), das 20 g/l Glucose enthält, wird wie in Beispiel 4 beimpft. Die Kultivierungsbedingungen sind ebenfalls wie in Beispiel 4. Nach 24-36 Stunden Fermentationsdauer und dem Verbrauch der gesamten Glucosemenge werden 6 g/l Harnstoff, 3 g/l Natriumacetat und 0,03 g/l CoCl₂ x 6 H₂O zudosiert. Nach dem Ansteigen des pH-Wertes des Mediums wird 30 %-ige Essigsäure zudosiert (fed-batch). Unter diesen Bedingungen gelang es, innerhalb von 72 Stunden, 19,6 g/l Biotrockenmasse mit einer spezifischen Aktivität von 315 U/mg herzustellen. Die Gesamtaktivität betrug 6174 U/ml.

### Beispiel 6:

Ein 15 I-Laborfermenter mit 7,0 I Medium "SI" (Zusammensetzung in g/l: Na₂HPO₄ x 12 H₂O -7,9; KH₂PO₄-1,8; MgSO₄ x 7 H₂O -1,0; Harnstoff - 4,0; Maisextrakt - 4,0; pH 7,2 ± 0,2), das 20 g/l Glucose enthält, wird mit 4 % (v/v) *Rhodococcus rhodochrous* M33-Inokulumkultur beimpft. Das Inokulum wurde innerhalb von 24 Stunden als Schüttelkultur in einem Medium gleicher Zusammensetzung angezüchtet.

Kultivierungsbedingungen:
- Temperatur 29 - 30°C
- Belüftungsrate 0,5 vvm
- Überlagerungsdruck 0,3 bar
- Rel. O₂-Partialdruck ≥ 50 % (über Rührerdrehzahl geregelt)
- Rührer-Drehzahl 300 - 600 min⁻¹

Nach 12 Stunden Fermentationsdauer (Beginn der exponentiellen Wachstumsphase) werden 0,01 g/l CoCl₂ x 6 H₂O zudosiert. Nach 21 bis 23 Stunden Fermentationsdauer und dem Verbrauch der gesamten Glucosemenge werden 6 g/l Harnstoff, 3 g/l Natriumacetat und 0,03 g/l CoCl₂ x 6 H₂O zudosiert. Nach dem Ansteigen des pH-Wertes der Kulturbrühe auf pH 7,9 erfolgt die Zudosierung der neuen Kohlenstoffquelle, Essigsäure (30 %-ig), über die pH-Regelung auf pH 7,9 (pH-stat feeding). Unter diesen Bedingungen gelang es, innerhalb von nur 66,5 Stunden, 19,9 g/l Biotrockenmasse mit einer spezifischen Aktivität von 294 U/mg herzustellen. Die Gesamtaktivität betrug 5851 U/ml.

Dadurch, dass CoCl₂ x 6 H₂O erstmals zu Beginn der exponentiellen Wachstumsphase dem Medium beigemischt wird, konnte gegenüber Beispiel 5 die Fermentationsdauer verkürzt werden.

### Beispiel 7:

Ein industrieller 15 m³-Produktionsfermenter mit 10 m³ Kultivierungsmedium "SI" (Zusammensetzung in g/l: H₃PO₄ - 3,46; KOH - 0,74; NaOH - zur Einstellung auf pH 6,9; CoCl₂ x 6 H₂O - 0,01; MgSO₄ x 7 H₂O - 1,0; Harnstoff - 4,0; Maisextrakt - 4,0; pH 7,2 ± 0,2), das 20 g/l Glucose enthält, wird mit 5 % (v/v) *Rhodococcus rhodochrous* M33-Inokulumkultur beimpft. Die Inokulumanzucht wurde vorab, innerhalb von 24 Stunden, in einem Vorfermenter ebenfalls mit Medium "SI" durchgeführt.

Kultivierungsbedingungen:
- Temperatur 28 - 30°C
- Rührer-Drehzahl 160 - 165 min⁻¹
- Belüftungsrate 0,5 vvm

Nach 24 bis 36 Stunden und nach dem Verbrauch der gesamten Glucosemenge werden dem Medium 6 g/l Harnstoff, 3 g/l Natriumacetat und 0,03 g/l CoCl₂ x 6 H₂O zudosiert. Das Feeding der Essigsäure erfolgt analog Beispiel 6. Nach 72 Stunden Fermentationsdauer gelang es, 18,8 g/l Biotrockenmasse mit einer spezifischen Aktivität von 290 U/mg herzustellen. Die Gesamtaktivität betrug 5452 U/ml.

## Patentansprüche

1. Verfahren zur Kultivierung des Nitrilhydratase-produzierenden Stammes *Rhodococcus rhodochrous* M33, der unter der Hinterlegungsbezeichnung DSM 14230 bei der DSMZ, Deutsche Sammlung von Mikroorganismen und Zellkulturen GmbH, Marschroder Weg 1 b, D-38124 Braunschweig, Deutschland, hinterlegt ist, **dadurch gekennzeichnet, dass** ein Kultivierungsmedium verwendet wird, das auf einem 12 bis 60 mM Phosphat-Puffer basiert und den Bedarf der Zellen an Phosphor deckt sowie den pH-Wert während der Kultivierung im Bereich von 5,5 bis 9,0 aufrecht erhält und in dem nach dem Verbrauch der vorgelegten Glucosemenge Essigsäure und/oder eines ihrer wasserlöslichen Salze als neue Kohlenstoffquelle zudosiert wird, wobei dem Kultivierungsmedium Harnstoff und CoCl₂ x 6 H₂O zudosiert werden.

2. Verfahren nach Anspruch 1, **dadurch gekennzeichnet, dass** in dem Kultivierungsmedium Maisextrakt als Wachstumsfaktor verwendet wird.

3. Verfahren nach Anspruch 1 oder 2, **dadurch gekennzeichnet, dass** als Maisextrakte Maisquellwasser und/oder Corn Steep-Lösung verwendet wird.

4. Verfahren nach einem der Ansprüche 1 bis 3, **dadurch gekennzeichnet, dass** der Maisextrakt in einer Konzentration von 1 bis 10 g/l im Kultivierungsmedium eingesetzt wird.

5. Verfahren nach einem der Ansprüche 1 bis 4, **dadurch gekennzeichnet, dass** als Kohlenstoffquelle im Kultivierungsmedium Glucose und Essigsäure eingesetzt werden.

6. Verfahren nach einem der Ansprüche 1 bis 5, **dadurch gekennzeichnet, dass** das Kultivierungsmedium als Kohlenstoffquelle Glucose in einer Einsatzmenge von 20 bis 90 g/l enthält.

7. Verfahren nach einem der Ansprüche 1 bis 6, **dadurch gekennzeichnet, dass** die Zugabe der Essigsäure zunächst in Form ihrer wasserlöslichen Salze erfolgt.

8. Verfahren nach einem der Ansprüche 1 bis 7, **dadurch gekennzeichnet, dass** als wasserlösliches Salz der Essigsäure Natriumacetat eingesetzt wird.

9. Verfahren nach einem der Ansprüche 1 bis 8, **dadurch gekennzeichnet, dass** das wasserlösliche Salz der Essigsäure zunächst vorgelegt und nach Anstieg des pH-Wertes auf pH 7,5 bis 8,5 Essigsäure zudosiert wird.

10. Verfahren nach einem der Ansprüche 1 bis 9, **dadurch gekennzeichnet, dass** das wasserlösliches Salz der Essigsäure in einer Einsatzmenge von 1 bis 4 g/l, vorzugsweise 3 g/l nach dem Verbrauch der Glucose zudosiert wird.

11. Verfahren nach einem der Ansprüche 1 bis 10, **dadurch gekennzeichnet, dass** die weitere Zugabe der Essigsäure in Form einer 20 bis 50 %-igen, vorzugsweise 30 %-igen wässrigen Lösung erfolgt.

12. Verfahren nach einem der Ansprüche 1 bis 11, **dadurch gekennzeichnet, dass** die Essigsäure-Lösung über ein pH-Stat-Feeding bei pH 7,5 bis 8,5, vorzugsweise bei pH 7. 9 zudosiert wird.

13. Verfahren nach einem der Ansprüche 1 bis 12, **dadurch gekennzeichnet, dass** Harnstoff als Induktor im Medium vorgelegt, und nach dem Verbrauch der vorgelegten Glucose nochmals zudosiert wird.

14. Verfahren nach einem der Ansprüche 1 bis 13, **dadurch gekennzeichnet, dass** CoCl₂ × 6 H₂O zu Beginn der exponentiellen Wachstumsphase, die auf der Kohlenstoffquelle Glucose basiert, dem Medium beigemischt wird und nach dem Verbrauch der Glucose nochmals zudosiert wird.

15. Verfahren nach einem der Ansprüche 1 bis 14, **dadurch gekennzeichnet, dass** bei der Kultivierung im Fermenter die Sauerstoffversorgung so gestaltet wird, dass der relative 0₂-Partialdruck grösser 30 %, vorzugsweise ≥ 50 % ist.

16. Verwendung eines Kultivierungsmediums bestehend aus
a.) 7,9 g/l Na₂HPO₄ × 12 H₂O
b.) 1,8 g/l KH₂PO₄
c.) 0,02 - 0,04 g/l CoCl₂ × 6 H₂O
d.) 1,0 g/l MgSO₄ × 7 H₂O
e.) 0 bis 10 g/l Maisextrakt
f.) 20 bis 90 g/l Glucose und
g.) 4 bis 20 g/l Harnstoff
in dem Verfahren nach einem der Ansprüche 1 bis 15.

17. Verwendung nach Anspruch 16, **dadurch gekennzeichnet, dass** das Kultivierungsmedium den Maisextrakt in einer Konzentration von 1 bis 10 g/l im Kultivierungsmedium enthält.

18. Verwendung nach einem der Ansprüche 16 oder 17, **dadurch gekennzeichnet, dass** es auf einem 12 bis 60 mM Phosphat-Puffer basiert und den Bedarf der Zellen an Phosphor deckt sowie den pH-Wert während der Kultivierung im Bereich von 5,5 bis 9,0 aufrecht erhält.

19. Verwendung nach einem der Ansprüche 16 bis 18, **dadurch gekennzeichnet, dass** das Medium Essigsäure als weitere Kohlenstoffquelle aufweist.

## Claims

1. Method for culturing the nitrile hydratase-producing strain *Rhodococcus rhodochrous* M33, which is deposited under the deposit name DSM 14230 at DSMZ, Deutsche Sammlung von Mikroorganismen und Zellkulturen GmbH [German Collection of Microorganisms and Cell Cultures], Marschroder Weg 1 b, D-38124 Braunschweig, Germany, **characterized in that** a culture medium is used which is based on a 12 to 60 mM phosphate buffer and covers the requirement for phosphorus of the cells, and also maintains the pH during the cultivation in the range from 5.5 to 9.0 and in which, following the consumption of the amount of glucose initially introduced, acetic acid and/or one of its water-soluble salts is metered in as a new source of carbon, where urea and CoCl₂ × 6 H₂O are metered into the culture medium.

2. Method according to Claim 1, **characterized in that** corn extract is used as growth factor in the culture medium.

3. Method according to Claim 1 or 2, **characterized in that** corn steep liquor and/or corn steep solution is used as corn extracts.

4. Method according to one of Claims 1 to 3, **characterized in that** the corn extract is used in the culture medium in a concentration of from 1 to 10 g/l.

5. Method according to one of Claims 1 to 4, **characterized in that** glucose and acetic acid are used as the source of carbon in the culture medium.

6. Method according to one of Claims 1 to 5, **characterized in that** the culture medium comprises glucose as the source of carbon in a use amount of from 20 to 90 g/l.

7. Method according to one of Claims 1 to 6, **characterized in that** the addition of the acetic acid takes place firstly in the form of its water-soluble salts.

8. Method according to one of Claims 1 to 7, **characterized in that** sodium acetate is used as the water-soluble salt of acetic acid.

9. Method according to one of Claims 1 to 8, **characterized in that** the water-soluble salt of acetic acid is initially introduced and, after increasing the pH to pH 7.5 to 8.5, acetic acid is metered in.

10. Method according to one of Claims 1 to 9, **characterized in that** the water-soluble salt of acetic acid is metered in in a use amount of from 1 to 4 g/l, preferably 3 g/l, following consumption of the glucose.

11. Method according to one of Claims 1 to 10, **characterized in that** the further addition of the acetic acid takes place in the form of a 20 to 50% strength, preferably 30% strength, aqueous solution.

12. Method according to one of Claims 1 to 11, **characterized in that** the acetic acid solution is metered in via a pH-stat feeding at pH 7.5 to 8.5, preferably at pH 7.9.

13. Method according to one of Claims 1 to 12, **characterized in that** urea is initially introduced in the medium as inductor, and is metered in again following the consumption of the initially introduced glucose.

14. Method according to one of Claims 1 to 13, **characterized in that** CoCl₂ × 6 H₂O is mixed into the medium at the start of the exponential growth phase, which is based on the carbon source glucose, and is metered in again following the consumption of the glucose.

15. Method according to one of Claims 1 to 14, **characterized in that** during the cultivation in the fermenter the oxygen supply is such that the relative O₂ partial pressure is greater than 30%, preferably ≥ 50%.

16. Use of a culture medium consisting of
a.) 7.9 g/l Na₂HPO₄ × 12 H₂O
b.) 1.8 g/l KH₂PO₄
c.) 0.02-0.04 g/l CoCl₂ × 6 H₂O
d.) 1.0 g/l MgSO₄ × 7 H₂O
e.) 0 to 10 g/l corn extract
f.) 20 to 90 g/l glucose and
g.) 4 to 20 g/l urea
in the method according to one of Claims 1 to 15.

17. Use according to Claim 16, **characterized in that** the culture medium comprises the corn extract in a concentration of from 1 to 10 g/l in the culture medium.

18. Use according to one of Claims 16 or 17, **characterized in that** it is based on a 12 to 60 mM phosphate buffer and covers the requirement for phosphorus of the cells, and also maintains the pH during the cultivation in the range from 5.5 to 9.0.

19. Use according to one of Claims 16 to 18, **characterized in that** the medium has acetic acid as a further source of carbon.

## Revendications

1. Procédé pour la culture de la souche *Rhodococcus rhodochrous* M33 productrice de nitrile hydratase, qui est déposée sous le numéro de dépôt DSM 14230 à la DSMZ, Deutsche Sammlung von Mikroorganismen und Zellkulturen GmbH, Marschroder Weg 1 b, D-38124,Braunschweig, Allemagne, **caractérisé en ce qu'**on utilise un milieu de culture qui est à base d'un tampon phosphate 12 à 60 mM et couvre le besoin des cellules en phosphore et pendant la culture on maintient le pH dans l'intervalle de 5,5 à 9,0 et dans lequel on ajoute par addition dosée, en fonction de la consommation de la quantité de glucose disposée au préalable, de l'acide acétique et/ou l'un de ses sels solubles dans l'eau, en tant que nouvelle source de carbone, de l'urée et CoCl₂ × 6 H₂O étant ajoutés par addition dosée au milieu de culture.

2. Procédé selon la revendication 1, **caractérisé en ce que** dans le milieu de culture on utilise de l'extrait de maïs en tant que facteur de croissance.

3. Procédé selon la revendication 1 ou 2, **caractérisé en ce qu'**on utilise comme extraits de maïs de la liqueur de trempage du maïs et/ou une solution de Corn Steep.

4. Procédé selon l'une quelconque des revendications 1 à 3, **caractérisé en ce que** l'extrait de maïs est utilisé à une concentration de 1 à 10 g/l dans le milieu de culture.

5. Procédé selon l'une quelconque des revendications 1 à 4, **caractérisé en ce qu'**on utilise comme source de carbone dans le milieu de culture du glucose et de l'acide acétique.

6. Procédé selon l'une quelconque des revendications 1 à 5, **caractérisé en ce que** le milieu de culture contient comme source de carbone du glucose en une quantité initiale de 20 à 90 g/l.

7. Procédé selon l'une quelconque des revendications 1 à 6, **caractérisé en ce que** l'addition de l'acide acétique s'effectue d'abord sous forme de ses sels solubles dans l'eau.

8. Procédé selon l'une quelconque des revendications 1 à 7, **caractérisé en ce qu'**on utilise comme sel hydrosoluble de l'acide acétique l'acétate de sodium.

9. Procédé selon l'une quelconque des revendications 1 à 8, **caractérisé en ce que** d'abord on dispose au préalable le sel hydrosoluble de l'acide acétique et, après élévation du pH à pH 7,5 à 8,5, on ajoute par addition dosée de l'acide acétique.

10. Procédé selon l'une quelconque des revendications 1 à 9, **caractérisé en ce que** le sel hydrosoluble de l'acide acétique est ajouté en une quantité initiale de 1 à 4 g/l, de préférence de 3 g/l après la consommation du glucose.

11. Procédé selon l'une quelconque des revendications 1 à 10, **caractérisé en ce que** l'addition ultérieure de l'acide acétique s'effectue sous forme d'une solution aqueuse à 20-50 %, de préférence à 30 %.

12. Procédé selon l'une quelconque des revendications 1 à 11, **caractérisé en ce que** la solution d'acide acétique est ajoutée par addition dosée au moyen d'une alimentation par pH-Stat à pH 7,5 - 8,5, de préférence à pH 7,9.

13. Procédé selon l'une quelconque des revendications 1 à 12, **caractérisé en ce qu'**on dispose au préalable de l'urée en tant qu'inducteur dans le milieu, et, après la consommation du glucose disposé au préalable, on en ajoute encore par addition dosée.

14. Procédé selon l'une quelconque des revendications 1 à 13, **caractérisé en ce qu'**on ajoute au milieu du CoCl₂ × 6 H₂O au début de la phase exponentielle de croissance, qui est basée sur la source de carbone glucose, et après la consommation du glucose on en ajoute encore par addition dosée.

15. Procédé selon l'une quelconque des revendications 1 à 14, **caractérisé en ce que** dans la culture dans le fermenteur l'alimentation en oxygène est faite de manière à ce que la pression partielle relative d'O₂ soit supérieure à 30 %, de préférence ≥ 50 %.

16. Utilisation d'un milieu de culture constitué de
a.) 7,9 g/l de Na₂HPO₄ × 12 H₂O
b.) 1,8 g/l de KH₂PO₄
c.) 0,02 - 0,04 g/l de CoCl₂ × 6 H₂O
d.) 1,0 g/l de MgSO₄ × 7 H₂O
e.) 0 à 10 g/l d'extrait de maïs
f.) 20 à 90 g/l de glucose et
g.) 4 à 20 g/l d'urée
dans le procédé selon l'une quelconque des revendications 1 à 15.

17. Utilisation selon la revendication 16, **caractérisée en ce que** le milieu de culture contient l'extrait de maïs à une concentration de 1 à 10 g/l dans le milieu de culture.

18. Utilisation selon l'une quelconque des revendications 16 ou 17, **caractérisée en ce que** le milieu de culture est à base d'un tampon phosphate 12 à 60 mM et couvre le besoin des cellules en phosphore et pendant la culture on maintient le pH dans l'intervalle de 5,5 à 9.

19. Utilisation selon l'une quelconque des revendications 16 à 18, **caractérisée en ce que** le milieu comporte de l'acide acétique en tant qu'autre source de carbone.
